**Europäisches Patentamt**

**European Patent Office**

**Office européen des brevets**

(19)

(11) Veröffentlichungsnummer: **0 323 840**
**A1**

# (12) EUROPÄISCHE PATENTANMELDUNG

(21) Anmeldenummer: **89100130.7**

(22) Anmeldetag: **05.01.89**

(51) Int. Cl.4: **C07C 19/02 , C07C 17/22**

(30) Priorität: **05.01.88 DE 3800122**

(43) Veröffentlichungstag der Anmeldung:
**12.07.89 Patentblatt 89/28**

(84) Benannte Vertragsstaaten:
**DE ES FR GB IT NL**

(71) Anmelder: **WACKER-CHEMIE GMBH**
**Prinzregentenstrasse 22**
**D-8000 München 22(DE)**

(72) Erfinder: **Schneider, Otto, Dr. Dipl.-Chem.**
**Stegerwaldstrasse 5**
**D-8263 Burghausen(DE)**
Erfinder: **Müller, Reinhardt, Dr. Dipl.-Chem.**
**Karl-Gros-Strasse 12**
**D-8263 Burghausen(DE)**

(54) **Spaltung von Dimethyläther.**

(57) Die Erfindung betrifft ein Verfahren zur Herstellung von Methylchlorid aus Dimethylether und Chlorwasserstoff an einem festen Zinkchlorid-Katalysator, wobei Chlorwasserstoff und Dimethylether im Molverhältnis von 2,0 zu 1 bis 2,5 zu 1 eingesetzt werden.

EP 0 323 840 A1

## Spaltung von Dimethylether

Bei der Herstellung von Methylchlorid aus Methanol und Chlorwasserstoff fallen als Nebenprodukt beträchtliche Mengen an Dimethylether an, welche aus dem Produktgemisch abgetrennt und im allgemeinen vernichtet werden. Die vorliegende Erfindung betriff die Spaltung von Dimethylether zu Methylchlorid.

Verfahren zur Spaltung von Dimethylether zu Methylchlorid sind grundsätzlich bekannt. JP-A 56-127 324 (offengelegt am 06. Oktober 1981, Erfinder: Y. Kobayashi et al., Shinetsu Chemicals Co. Ltd.) beschreibt die Spaltung von Dimethylether an Zinkchlorid auf Aktivkohle als Katalysator, wobei drei bis vier Mol Chlorwasserstoff pro Mol Dimethylether eingesetzt werden, um gute Ausbeuten an Methylchlorid zu erzielen. Um die Menge an zu deponierender Salzsäure gering zu halten, wird dort in einem zweiten Schritt die entstandene Salzsäure mit Methanol zu Methylchlorid umgesetzt. Eine Ausbeute von 89 % erzielt W. Sundermann (Chemische Berichte 97, Seite 1069 - 1074 (1966)) an in einer Kaliumchloridschmelze gelöstem Zinkchlorid, wobei 2,2 bis 2,5 Mol Chlorwasserstoff pro Mol Dimethylether eingesetzt werden.

Aufgabe der vorliegenden Erfindung war es, an einem preiswerten Katalysator aus Dimethylether in hoher Ausbeute Methylchlorid herzustellen. Eine weitere Aufgabe war, bei diesem Verfahren den Anfall an zu deponierenden Komponenten im Produktgemisch gering zu halten. Es war weiterhin Aufgabe der vorliegenden Erfindung, Methylchlorid in hoher Reinheit bzw. in leicht zu reinigender Form herzustellen.

Diese Aufgaben werden durch die vorliegende Erfindung dadurch gelöst, daß Dimethylether und Chlorwasserstoff an einem festen Zinkchlorid-Katalysator umgesetzt werden, welcher vorzugsweise aus auf Aktivkohle aufgebrachtem Zinkchlorid besteht, wobei Chlorwasserstoff und Dimethylether im Molverhältnis 2,0 zu 1 bis 2,5 zu 1, vorzugsweise 2,05 zu 1 bis 2,2 zu 1, eingesetzt werden.

Die Reaktionspartner werden vorzugsweise bei einer Temperatur von $120\,^\circ$C bis $350\,^\circ$C, insbesondere von $150\,^\circ$C bis $250\,^\circ$C, speziell von $180\,^\circ$C bis $220\,^\circ$C mit dem festen Zinkchlorid-Katalysator in Kontakt gebracht.

Das Verfahren kann beim Druck der umgebenden Atmosphäre, also ca. 0,1 MPa (abs.), es kann auch bei höheren oder bei niedrigeren Drücken durchgeführt werden. Drucke von 0,09 MPa (abs.) bis 0,6 MPa (abs.) sind bevorzugt.

Das Verfahren beruht auf der chemischen Reaktion gemäß folgender Gleichung:

$$H_3C-O-CH_3 + 2HCl \longrightarrow 2CH_3Cl + H_2O$$

Da also bei der Reaktion auch Wasser entsteht, führt ein Überschuß an HCl zum Anfall von Salzsäure. Dies ist aber unerwünscht, da die Salzsäure sich in den meisten Fällen nicht gewinnbringend weiterverarbeiten läßt und deshalb i. a. entsorgt werden muß. Es war angesichts des Standes der Technik unerwartet, daß ohne bzw. mit einem niedrigem Über schuß von HCl Dimethylether in guten Ausbeuten gespalten werden konnte. Gemäß den untenstehenden Beispielen wurden jeweils Ausbeuten von über 98 % erreicht. Das Verfahren kann auch mit verunreinigtem Dimethylether durchgeführt werden. Insbesondere Verunreinigungen durch Methylchlorid (siehe Beispiel 4) und durch Organosiliciumverbindungen, speziell cyclische Dimethylsiloxane, und durch Wasser (siehe Beispiel 5) stören nicht.

Das Verfahren kann absatzweise, teilkontinuierlich oder kontinuierlich durchgeführt werden. Vorzugsweise wird es kontinuierlich durchgeführt.

Die Reaktionsbedingungen sind vorzugsweise so zu wählen, daß die Reaktionspartner (Dimethylether, HCl) und auch die (weiteren) im Produktgemisch vorliegenden Stoffe (Methylchlorid, Wasser) unter den am Katalysator herrschenden Bedingungen gasförmig sind. Die Reaktionspartner werden vorzugsweise als Gase in den Reaktor zugegeben. Die Reaktionspartner konnen durch ein Gas, wie Stickstoff, verdünnt werden. Es ist jedoch bevorzugt, die Reaktionspartner unverdünnt einzusetzen.

Das erfindungsgemäße Verfahren eignet sich besonders zur Herstellung von Methylchlorid aus dem bei der Darstellung von Methylchlorid aus Methanol als Nebenprodukt anfallendem Dimethylether. Zu diesem Zweck muß Dimethylether erst vom Methylchlorid abgetrennt werden. Dies gelingt insbesondere durch Absorption und anschließender Desorption des Dimethylethers in Wasser. Die Absorption wird vorzugsweise bei Temperaturen von $0\,^\circ$C bis $+30\,^\circ$C, besonders von $+5\,^\circ$C bis $+20\,^\circ$C, die Desorption vorzugsweise bei Normaldruck (0,1 MPa(abs.)) und Temperaturen von $70\,^\circ$C bis $110\,^\circ$C, besonders von $95\,^\circ$C bis $105\,^\circ$C durchgeführt. Nach der Absorption noch im Produktgemisch verbliebener Dimethylether kann, falls gewünscht, mit Schwefelsäure extrahiert werden. Der so durch Absorption und Desorption abgetrennte Dimethylether wird nach dem erfindungsgemäßen Verfahren zu Methylchlorid umgesetzt und dieses Methylchlorid mit dem oben genannten, vom Dimethylether weitgehend befreiten Produktgemisch, vereinigt. Im Laufe des erfindungsgemäßen Verfahrens werden vorzugsweise die Reaktionspartner, nämlich Chlorwasserstoff und Dimethylether, gegebenenfalls nach vorheriger Erwärmung, in einem indirekt beheizten Festbettreaktor, dessen Festbett den festen Zinkchlorid-Katalysator enthält, mit diesem in Kontakt gebracht, und das so erhaltene Produktgemisch auf eine Temperatur zwischen dem Siedepunkt des Methylchlorids und dem der entstandenen Salzsäure abgekühlt, wobei die Salzsäure vom Produktgemisch abgetrennt wird.

Als Festbettreaktor wird insbesondere ein mit einer festen Katalysator enthaltenden Füllung beschickter, mit einem fluiden Wärmeträger beheizter Rohrbündelreaktor eingesetzt.

Als fluider Wärmeträger werden vorzugsweise entsprechend temperierte Gase oder Flüssigkeiten, wie gegebenenfalls gespannter Wasserdampf oder Wärmeübertragungsöle eingesetzt. Eine gleichmäßige Temperaturverteilung im Reaktor ist im erfindungsgemäßen Verfahren von Vorteil. Die Reaktionswärme sollte weitgehend abgeführt werden, wobei die Abwärme zur Erzeugung von Wasserdampf eingesetzt werden kann. Als Katalysatoren sind Stränge bevorzugt, welche 10 bis 50, insbesondere 20 bis 40 Gewichtsprozent Zinkchlorid enthalten.

In den nachfolgenden Beispielen wurden Stränge mit 30 Gew.% Zinkchlorid eingesetzt. Der restliche Anteil der Stränge bestand überwiegend aus Aktivkohle.

Beispiele:

In einem mit Wärmeträgeröl thermostatisierten Rohrbündelreaktor aus Glas wurde jeweils eine Mischung aus Dimethylether (gegebenenfalls mit Verunreinigungen) und Chlorwasserstoff, im oberen, nicht mit Katalysator gefüllten Teil des Reaktors aufgeheizt, beim Druck der umgebenden Atmosphäre (0,1 MPa (abs.)) durch ein Bett mit 200 ml (100 g) Katalysator geleitet und nach Austritt aus dem Reaktor in einem Glaskühler auf + 5 bis + 10°C abgekühlt und die kondensierte Salzsäure abgetrennt.

In den Beispielen 6 und 7 (Vergleichsbeispiele) wurde statt dem Rohrbundelreaktor ein mit Wärmeträgeröl indirekt beheiztes Doppelmantelglasrohr (Höhe 0,4 m, Innendurchmesser: 25 mm) das bis zur halben Höhe mit 100 ml des oben beschriebenen Katalysators beschickt war, verwendet. Im Doppelmantelglasrohr wird eine im Vergleich zum Rohrbündelreaktor ungleichmäßigere Temperaturverteilung erzielt. Tabelle 1 führt die erfindungsgemäßen Beispiele 1 - 5, Tabelle 2 die Vergleichsbeispiele 6 - 10 auf.

Tabelle 1

| Erfindungsgemäße Beispiele | | | | | | |
|---|---|---|---|---|---|---|
| Beispiel | HCl:DME :MeCl (Mol) | Reaktortemperatur (°C) | 1 Gas eingespeist pro 1 Kat . h | DME im erzeugten MeCl (Gew.%) | HCl in MeCl (Gew.%) | HCl in wässrigem Kondensat (Gew.%) |
| 1 | 2,15:1:0 | 200 | 830 | 0,20 | $n^3$ | 26,6 |
| 2 | 2,09:1:0 | 200 | 770 | 0,11 | $n^3$ | 22,0 |
| 3 | 2,05:1:0 | 200 | 750 | 1,0 | 0,1 | 20,4 |
| 4 | 2,1:1:1 | 200 | 1000 | 1,1 | 0,1 | $n^3$ |
| 5 | 2,1:1 :0 | 200 | 750 | 1,0 | $n^3$ | 24,0 |

1) DME : Dimethylether

2) MeCl: Methylchlorid

3) n: nicht bestimmt

4) DME wurde bei Raumtemperatur durch $((CH_3)_2SiO)_4$ geleitet und somit ein siloxanhaltiges Gasgemisch in den Reaktor geleitet.

EP 0 323 840 A1

Tabelle 2

| Nicht erfindungsgemäße Beispiele | | | | | |
|---|---|---|---|---|---|
| Beispiel | Reaktortyp | HCl:DME[1]:MeCl[2] (Mol) | Reaktortemp. (°C) | 1 Gas eingesetzt pro 1 Kat . h | DME[1] im erzeugten MeCl[2] bezogen auf MeCl (Gew.%) |
| 6 | 5) | 3:1:0 | 150 | 1000 | 6,6 |
| 7 | 5) | 3:1:0 | 200 | 1000 | 4,1 |
| 8 | 6) | 3:1:0 | 160 | 1500 | 0,65 |
| 9 | 6) | 3:1:0 | 200 | 1500 | 0,12 |
| 10 | 6) | 3:1:3 | 200 | 1160 | 0,15 |
| Durch den hohen HCl-Überschuß enthält das entstehende MeCl (nach Kühlung auf ca. 5° C) noch erhebliche Mengen HCl. | | | | | |

1) Doppelmantelgasrohr
2) wie in den Beispielen 1-5

## Ansprüche

1. Verfahren zur Herstellung von Methylchlorid aus Dimethylether und Chlorwasserstoff an einem festen Zinkchlorid-Katalysator, dadurch gekennzeichnet, daß Chlorwasserstoff und Dimethylether im Molverhältnis von 2,0 zu 1 bis 2,5 zu 1 eingesetzt werden

2. Verfahren nach Anspruch , wobei Chlorwasserstoff und Dimethylether im Molverhältnis von 2,05 zu 1 bis 2,2 zu eingesetzt werden.

3. Verfahren nach Anspruch 1 oder 2, wobei der feste Zinkchlorid-Katalysator aus auf Aktivkohle aufgebrachtem Zinkchlorid besteht.

4. Verfahren nach einem der Ansprüche 1, 2 oder 3, wobei die Reaktionspartner bei einer Temperatur von 120° C bis 350° C, vorzugsweise 150° C bis 250° C mit dem festen Zinkchlorid-Katalysator in Kontakt gebracht werden.

5. Verfahren nach einem der Ansprüche 1, 2, 3 oder 4, wobei die Reaktionspartner bei einem Druck von 0,09 MPa bis 0,6 MPa (abs.) mit dem festen Zinkchlorid-Katalysator in Kontakt gebracht werden.

6. Verfahren nach einem der Ansprüche 1, 2, 3, 4 oder 5, welches kontinuierlich durchgeführt wird.

7. Kontinuierliches Verfahren nach Anspruch 6, wobei Chlorwasserstoff und Dimethylether, gegebenenfalls nach vorheriger Erwärmung, in einem indirekt beheizten Festbettreaktor, dessen Festbett den festen Zinkchlorid-Katalysator enthalt, mit diesem in Kontakt gebracht werden, und das so erhaltene Produktgemisch auf eine Temperatur zwischen dem Siedepunkt des Methylchlorids und dem der entstandenen Salzsäure abgekühlt wird, wobei die Salzsäure vom Produktgemisch abgetrennt wird.

8. Kontinuierliches Verfahren nach Anspruch 7, wobei als Festbettreaktor ein mit einer festen Katalysator enthaltenden Füllung beschickter, mit einem fluiden Wärmeträger beheizter Rohrbündelreaktor eingesetzt wird.

9. Verfahren zum Reinigen und Erhöhen der Ausbeute in einem Produktgemisch enthaltend Methylchlorid als gewünschtes und Dimethylether als unerwünschtes Produkt, wobei man Dimethylether durch Absorption und anschließender Desorption in Wasser aus dem Produktgemisch abtrennt, dem Verfahren nach einem der Ansprüche 1 bis 8 unterzieht, und das so hergestellte Methylchlorid mit dem oben genannten, von Dimethylether befreiten Produktgemisch vereinigt.

10. Verfahren nach einem der Ansprüche 1 bis 9, wobei der eingesetzte Dimethylether verunreinigt ist, insbesondere durch Methylchlorid und/oder Organosiliciumverbindungen und/oder Wasser.

5

**Europäisches Patentamt**

**EUROPÄISCHER RECHERCHENBERICHT**

Nummer der Anmeldung

EP 89100130.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | <u>EP - A1 - 0 136 549</u> (HENKEL)<br><br> * Ansprüche 1,4-8; Beispiel 1 *<br><br>-- | 1,2,4-6,10 | C 07 C 19/02<br>C 07 C 17/22 |
| A | <u>GB - A - 1 134 116</u> (ICI)<br><br> * Ansprüche 12,16-18; Beispiel 4 *<br><br>-- | 1,2,4-6 | |
| D,A | <u>JP - A - 56-127 324</u> (SHINETSU)<br><br> * Anspruch; Beispiel 1 *<br><br>-- | 1,3-6 | |
| A | SOVIET INVENTIONS ILLUSTRATED, Sektion I, 17. Jänner 1974, Woche U50<br><br>DERWENT PUBLICATIONS LTD., London, E 16<br><br> * SU-375 279 (A.P. MANTULO et al.) *<br><br>---- | 1,3-5 | |

| RECHERCHIERTE SACHGEBIETE (Int. Cl.4) |
|---|
| C 07 C 19/00 |
| C 07 C 17/00 |

Der vorliegende Recherchenbericht wurde für alle Patentansprüche erstellt.

| Recherchenort | Abschlußdatum der Recherche | Prüfer |
|---|---|---|
| WIEN | 23-03-1989 | KÖRBER |